# EUROPEAN PATENT SPECIFICATION

(11) **EP 1 366 783 B1**
(45) Date of publication and mention of the grant of the patent: **05.04.2006**
(21) Application number: 02011863.4
(22) Date of filing: 28.05.2002
(51) Int. Cl.: A61P 31/02, A61K 36/00, A61K 9/00

(54) **Medicinal herbal preparation for disinfecting vaginas**
Medizinische herbale Zusammensetzung zur vaginalen Desinfektion
Composition médicinale herbale pour la désinfection vaginale

(43) Date of publication of application: 03.12.2003
(73) Proprietor: CHEN, Bih Cheng, Taichung (TW)
(72) Inventor: CHEN, Bih Cheng, Taichung (TW)
(74) Representative: Zeitler, Giselher

(56) References cited:
- WO-A-92/19320
- FR-A- 2 775 595
- US-A- 5 837 254
- US-A- 6 027 728
- DATABASE WPI Week 200310 Derwent Publications Ltd., London, GB; AN 2003-104122 XP002236021 & CN 1 188 007 A (HE Y), 22 July 1998 (1998-07-22)
- DATABASE WPI Week 200043 Derwent Publications Ltd., London, GB; AN 2000-483367 XP002236022 & CN 1 253 828 A (YANG X), 24 May 2000 (2000-05-24)

## Description

### BACKGROUND OF THE INVENTION

### 1. Field of the Invention

The present invention relates to a medicinal preparation, and more particularly to a medicinal preparation for cleaning and disinfecting and moistening and tightening the vaginas of women.

### 2. Description of the Prior Art

Typically, the vaginas of women may have flaccid muscles, or the mucous membranes of the vaginas may become thinner and weak or fragile, or women may feel dry and scorching hot in the vaginas, particularly after childbirth, sexual intercourse, or when women have endocrine disorders. In addition, due to personal hygienic problems, or owing to the infection by germs or bacteria, the vaginas of women may have bad odors, inflammation, leucorrhoea, or the women may feel itchy in the vaginas, and thus may lose sexuality. Various kinds of medicinal preparations have been developed for cleaning the vaginas, for disinfecting the vaginas, for moistening the vaginas, or for tightening the vaginas separately. However, the medicinal preparations may not be used for cleaning and disinfecting and moistening and tightening the vaginas of women simultaneously.

The present invention has arisen to mitigate and/or obviate the afore-described disadvantages of the conventional medicinal preparations.

### SUMMARY OF THE INVENTION

The primary objective of the present invention is to provide a medicinal preparation for tightening the vaginas of women, or for increasing or recovering the resilience of the muscles of the vaginas of the women.

The other objective of the present invention is to provide a medicinal preparation for disinfecting the vaginas and to remove the bad odors, inflammation, leucorrhoea or the like from the vaginas of women.

The further objective of the present invention is to provide a medicinal preparation for cleaning and moistening the vaginas of women, for avoiding the vaginas infectious problems.

In accordance with one aspect of the invention, there is provided a medicinal preparation comprising a plurality of medicinal materials including asari radix, cuidii fructns, nelumbo nucifera gaertner, ginseng radix, angelica sinensis radix, scutellariae baicalennsis, phellodendron amurense radix, evodiae fructus, and saussureae radix, the medicinal materials being grounded into powder, and water blending with the powder of the medicinal materials to form a pasty material, in order to clean or to moisten or tighten the vaginas of women, or to increase or recover the resilience of the muscles of the vaginas of the women, or to disinfect the vaginas of women, to remove the bad odors, inflammation, leucorrhoea or the like from the vaginas of women, or to clean the vaginas of women, or to avoid the vaginas infectious problems.

The asari radix includes 10-30% by weight, the cnidii fructus includes 10-30% by weight, the nelumbo nucifera gaertner includes 5-10% by weight, the ginseng radix includes 3-5% by weight, the angelica sinensis radix includes 5-10% by weight, the scutellariae baicalennsis includes 5-10% by weight, the phellodendron amurense radix includes 5-10% by weight, the evodiae fructus includes 10-30% by weight, and the saussureae radix includes 10-30% by weight.

The pasty material may be refrigerated and dried to remove water from the pasty material and to refrigerate the pasty material to a crystallized material.

A plurality of substrates may then be mixed with the pasty material or with the crystallized material, the substrates include papaya enzyme, aloe jelly, lilly nature plant extractions, glycerine, almond oil, ginseng perfume, fatty acid, lactic acid, carbomer, and water material.

The papaya enzyme includes 10% by weight, the aloe jelly includes 10% by weight, the lilly nature plant extractions includes 0.5% by weight, the glycerine includes 15% by weight, the almond oil includes 15% by weight, the ginseng perfume includes 0.5% by weight, the fatty acid includes 5% by weight, the lactic acid includes 0.1% by weight, the carbomer includes 20% by weight, and the water material includes 43.9% by weight.

The substrates and the pasty material may then be rotated with a rotational speed of 1500 rps for about 20 minutes and for well mixing the substrates and the pasty material to form a gluey material.

Further objectives and advantages of the present invention will become apparent from a careful reading of a detailed description provided hereinbelow, with appropriate reference to accompanying drawings.

### BRIEF DESCRIPTION OF THE DRAWINGS

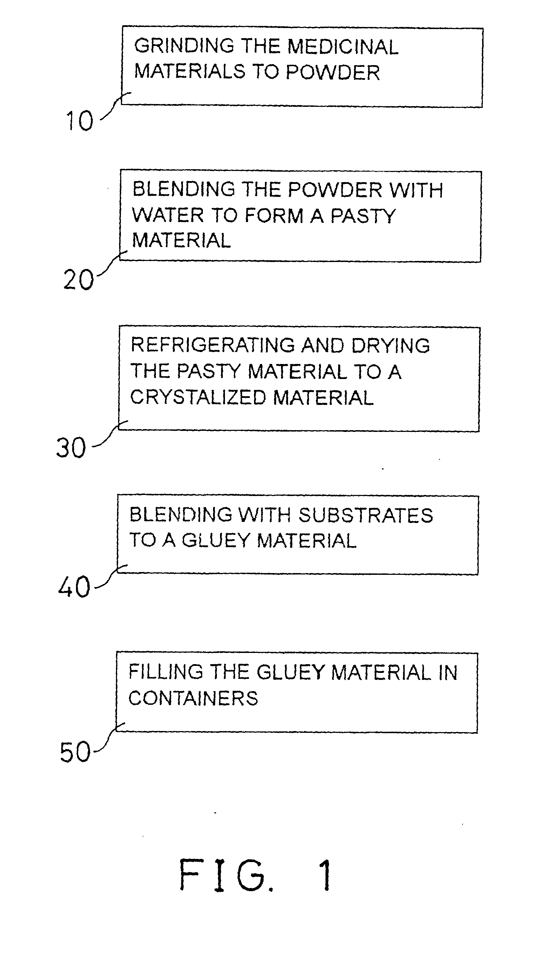

FIG. 1 is a block diagram illustrating the procedures for manufacturing a medicinal preparation in accordance with the present invention.

### DETAILED DESCRIPTION OF THE PREFERRED EMBODIMENT

Referring to the drawing, illustrated are the procedures for manufacturing a medicinal preparation in accordance with the present invention. First, a number of medicinal materials are required to be prepared, and may be used for moistening, nourishing, disinfecting, sterilizing, or for decreasing inflammation or the like. The medicinal materials include asari radix having 10-30% by weight, cnidii fructus having 10-30% by weight, nelumbo nucifera gaertner having 5-10% by weight, ginseng radix having 3-5% by weight, angelica sinensis radix having 5-10% by weight, scutellariae baicalennsnis having 5-10 % by weight, phellodendron amurense radix having 5-10% by weight, evodiae fructus having 10-30% by weight, and saussureae radix having 10-30% by weight.

The medicinal materials are medicinal herbs and are ground into powder or particles each having about 120 milligrams, or the like, in the process 10 as shown in FIG. 1. The powder is then blended with water or the other fluid, in order to form a pasty material, in the process 20 as shown in FIG. 1, for allowing the powder of the various medicinal herbs or materials to be suitably or well mixed or blended with each other. The pasty material is then subjected to a refrigerating and drying process 30 (FIG. 1) in order to dry the pasty material or to remove the water from the pasty material, and in order to decrease the temperature of the pasty material to about 10°C below zero degrees, or to refrigerate the pasty material into a crystallized material.

A number of substrates are then prepared and include papaya enzyme having 10% by weight, aloe jelly having 10% by weight, lilly nature plant extractions having 0.5% by weight, glycerine having 15% by weight, almond oil having 15% by weight, ginseng perfume having 0.5% by weight, fatty acid having 5% by weight, lactic acid having 0.1% by weight, distillation water or the typical water having 43.9% by weight, and carbomer having 20% by weight. The substrates are then mixed or blended with the crystallized material, and are rotated for a predetermined term of time, such as 20 minutes, and are rotated with a rotational speed of about 1500 rps, for allowing the substrates and the crystallized material to be suitably or uniformly mixed or blended with each other, and in order to form a sticky or gummy or gluey material in the process 40. The substrates may also be directly mixed with the pasty material, instead of the crystallized material.

The gluey material may then be filled into containers or envelopes or capsules in process 50 (FIG. 1) for being used by the users. For example, the medicinal preparation may be filled into the capsules and may then be taken orally, or may be directly applied onto the vaginas of the users, or the like. It is to be noted that the medicinal materials or the medicinal herbs are primarily used for medical use. The substrates may be selectively or optionally included or added into the medicinal materials or the medicinal herbs, but not necessarily be included or added into the medicinal materials or the medicinal herbs.

The medicinal preparation thus prepared include a number of medicinal herbs or materials that may be used for moistening, nourishing, disinfecting, sterilizing, or for decreasing inflammation or the like, such that the medicinal preparation may be used for moistening and tightening the vaginas of women or for increasing or recovering the resilience of the muscles of the vaginas of the women, or may be used for disinfecting the vaginas of women, in order to remove the bad odors, inflammation, leucorrhoea or the like from the vaginas of women, or may be used for cleaning and moistening the vaginas of women, for avoiding the vaginas infectious problems, and for hygienic use.

Accordingly, the medicinal preparation in accordance with the present invention may be used for moistening and tightening the vaginas of women, for increasing or recovering the resilience of the muscles of the vaginas of the women, or for disinfecting the vaginas of women, in order to remove the bad odors, inflammation, leucorrhoea or the like from the vaginas of women, or for cleaning and moistening the vaginas of women, for avoiding the vaginas infectious problems.

## Claims

1. A medicinal preparation comprising:
a plurality of medicinal materials including asari radix, cnidii fructus, nelumbo nucifera gaertner, ginseng radix, angelica sinensis radix, scutellariae baicalennsis, phellodendron amurense radix, evodiae fructus, and saussureae radix, said medicinal materials being ground into powder, and
water blending with said powder of said medicinal materials to form a pasty material.

2. The medicinal preparation according to claim 1, wherein said asari radix includes 10-30% by weight, said cnidii fructus includes 10-30% by weight, said nelumbo nucifera gaertner includes 5-10% by weight, said ginseng radix includes 3-5% by weight, said angelica sinensis radix includes 5-10% by weight, said scutellariae baicalennsis includes 5-10% by weight, said phellodendron amurense radix includes 5-10% by weight, said evodiae fructus includes 10-30% by weight, and said saussureae radix includes 10-30% by weight.

3. The medicinal preparation according to claim 1 further comprising a plurality of substrates mixed with said pasty material, said substrates including papaya enzyme, aloe jelly, lilly nature plant extractions, glycerine, almond oil, ginseng perfume, fatty acid, lactic acid, carbomer, and water material.

4. The medicinal preparation according to claim 3, wherein said papaya enzyme includes 10% by weight, said aloe jelly includes 10% by weight, said lilly nature plant extractions includes 0.5% by weight, said glycerine includes 15% by weight, said almond oil includes 15% by weight, said ginseng perfume includes 0.5% by weight, said fatty acid includes 5% by weight, said lactic acid includes 0.1% by weight, said carbomer includes 20% by weight, and said water material includes 43.9% by weight.

5. The medicinal preparation according to claim 3, wherein said substrates and said pasty material are rotated with a rotational speed of 1500 rps for well mixing said substrates and said pasty material to form a gluey material.

6. The medicinal preparation according to claim 1, wherein said pasty material is refrigerated and dried to remove water from said pasty material and to refrigerate said pasty material to a crystallized material.

7. The medicinal preparation according to claim 6 further comprising a plurality of substrates mixed with said crystallized material, said substrates including papaya enzyme, aloe jelly, lilly nature plant extractions, glycerine, almond oil, ginseng perfume, fatty acid, lactic acid, carbomer, and water material.

8. The medicinal preparation according to claim 7, wherein said papaya enzyme includes 10% by weight, said aloe jelly includes 10% by weight, said lilly nature plant extractions includes 0.5% by weight, said glycerine includes 15% by weight, said almond oil includes 15% by weight, said ginseng perfume includes 0.5% by weight, said fatty acid includes 5% by weight, said lactic acid includes 0.1% by weight, said carbomer includes 20% by weight, and said water material includes 43.9% by weight.

9. The medicinal preparation according to claim 7, wherein said substrates and said crystallized material are rotated with a rotational speed of 1500 rps for well mixing said substrates and said crystallized material to form a gluey material.

## Revendications

1. Composition médicinale, comprenant :
une pluralité de produits médicinaux qui incluent asari radix, cnidii fructus, nelumbo nucifera gaertner, ginseng radix, angelica sinensis radix, scutellariae baicalensis, phellodendron amurense radix, evodiae fructus, et saussureae radix, lesdits produits médicinaux étant broyés en poudre, et
de l'eau mélangée avec ladite poudre desdits produits médicinaux pour former un matériau pâteux.

2. Composition médicinale selon la revendication 1, dans laquelle ledit asari radix est inclus à raison de 10 à 30 % en poids, ledit cnidii fructus est inclus à raison de 10 à 30 % en poids, ledit nelumbo nucifera gaertner est inclus à raison de 5 à 10 % en poids, ledit ginseng radix est inclus à raison de 3 à 5 % en poids, ledit angelica sinensis radix est inclus à raison de 5 à 10 % en poids, ledit scutellariae baicalensis est inclus à raison de 5 à 10 % en poids, ledit phellodendron amurense radix est inclus à raison de 5 à 10 % en poids, ledit evodiae fructus est inclus à raison de 10 à 30 % en poids, et ledit saussureae radix est inclus à raison de 10 à 30 % en poids.

3. Composition médicinale selon la revendication 1, comprenant en outre une pluralité de substrats mélangés avec ledit matériau pâteux, lesdits substrats incluant de l'enzyme de papaye, du gel d'aloès, des extraits de plante naturelle de lys, de la glycérine, de l'huile d'amande, du parfum de ginseng, des acides gras, de l'acide lactique, du carbomer, et de l'eau.

4. Composition médicinale selon la revendication 3, dans laquelle ladite enzyme de papaye est incluse à raison de 10 % en poids, ledit gel d'aloès est inclus à raison de 10 % en poids, lesdits extraits de plante naturelle de lys sont inclus à raison de 0,5 % en poids, ladite glycérine est incluse à raison de 15 % en poids, ladite huile d'amande est incluse à raison de 15 % en poids, ledit parfum de ginseng est inclus à raison de 0,5 % en poids, ledit acide gras est inclus à raison de 5 % en poids, ledit acide lactique est inclus à raison de 0,1 % en poids, ledit carbomer est inclus à raison de 20 % en poids, est ladite eau est incluse à raison de 43,9 % en poids.

5. Composition médicinale selon la revendication 3, dans laquelle lesdits substrats et ledit matériau pâteux sont mis en rotation à une vitesse de rotation de 1500 tours par minute pour bien mélanger lesdits substrats et ledit matériau pâteux pour former un matériau gluant.

6. Composition médicinale selon la revendication 1, dans laquelle ledit matériau pâteux est réfrigéré et séché pour supprimer l'eau dudit matériau pâteux et pour réfrigérer ledit matériau pâteux en un matériau cristallisé.

7. Composition médicinale selon un la revendication 6, comprenant en outre une pluralité de substrats mélangés avec ledit matériau cristallisé, lesdits substrats incluant de l'enzyme de papaye, du gel d'aloès, des extraits de plante naturelle de lys, de la glycérine, de l'huile d'amande, du parfum de ginseng, des acides gras, de l'acide lactique, du carbomer, et de l'eau.

8. Composition médicinale selon la revendication 7, dans laquelle ladite enzyme de papaye est incluse à raison de 10 % en poids, ledit gel d'aloès est inclus à raison de 10 % en poids, lesdits extraits de plante naturelle de lys sont inclus à raison de 0,5 % en poids, ladite glycérine est incluse à raison de 15 % en poids, ladite huile d'amande est incluse à raison de 15 % en poids, ledit parfum de ginseng est inclus à raison de 0,5 % en poids, ledit acide gras est inclus à raison de 5 % en poids, ledit acide lactique est inclus à raison de 0,1 % en poids, ledit carbomer est inclus à raison de 20 % en poids, est ladite eau est incluse à raison de 43,9 % en poids.

9. Composition médicinale selon la revendication 7, dans laquelle lesdits substrats et ledit matériau cristallisé sont mis en rotation à une vitesse de rotation de 1500 tours par minute pour bien mélanger lesdits substrats et ledit matériau cristallisé pour former un matériau gluant.

## Patentansprüche

1. Arzneimittel zur Behandlung der Vagina, **gekennzeichnet durch** mehrere medizinische Stoffe, einschließlich Asariradix, Nesselfruktus, Nelumbonuciferagärtner, Ginsengwurzel, Angelicasinensisradix, Scutellariaebaicalennsis, Phellodendronamurenseradix, Evodiaefructus und Saussureaeradix, die zu Pulver zermahlen werden, wobei das Pulver dann mit Wasser zur Bildung eines pastösen Materials gemischt wird.

2. Arzneimittel nach Anspruch 1, **dadurch gekennzeichnet, daß** Asariradix mit 10-30 Gew.-%, Nesselfruktus mit 10 - 30 Gew.-%, Nelumbonuciferagärtner mit 5 - 10 Gew.-%, Ginsengwurzel mit 3 - 5 Gew.-%, Angelicasinensisradix mit 5 - 10 Gew.-%, Scutellariaebaicalennsis mit 5 - 10 Gew.-%, Phellodendronamurenseradix mit 5 -10 Gew.-%, Evodiaefructus mit 10 - 30 Gew.% und Saussureaeradix10 - 30 Gew.-% enthalten sind.

3. Arzneimittel nach Anspruch 1, **gekennzeichnet durch** mehrere Substrate, die mit dem pastösen Material vermischt sind und Papayaenzyme, Aloegelee, Liliennaturpflanzenextrakte, Glyzerin, Mandelöl, Ginsengparfüm, Fettsäure, Milchsäure, Carbomer und Wasser enthalten.

4. Arzneimittel nach Anspruch 3, **dadurch gekennzeichnet, daß** Papayaenzym mit 10 Gew.-%, Aloegelee mit 10 Gew.-%, Liliennaturpflanzenextrakte mit 0,5 Gew.-%, Glyzerin mit 15 Gew.-%, Mandelöl mit 15 Gew.-%, Ginsengparfüm mit 0,5 Gew.-%, Fettsäure mit 5 Gew.-%, Milchsäure mit 0,1 Gew.-%, Carbomer mit 20 Gew.% und Wasser mit 43,9 Gew.-% enthalten sind.

5. Arzneimittel nach Anspruch 3, **dadurch gekennzeichnet, daß** die Substrate und das pastöse Material mit einer Drehzahl von 1.500 Upm zwecks guter Vermischung der Substrate und des pastösen Materials zur Bildung eines Kleisters zentrifugiert werden.

6. Arzneimittel nach Anspruch 1, **dadurch gekennzeichnet, daß** das pastöse Material tiefgekühlt und getrocknet wird, um aus ihm Wasser zu entfernen, und daß das pastöse Material zur Bildung eines kristallinen Materials tiefgekühlt wird.

7. Arzneimittel nach Anspruch 6, **gekennzeichnet durch** mehrere Substrate, die mit dem kristallisierten Material vermischt werden und Papayaenzyme, Aloegelee, Liliennaturpflanzenextrakte, Glyzerin, Mandelöl, Ginsengparfüm, Fettsäure, Milchsäure, Carbomer und Wasser enthalten.

8. Arzneimittel nach Anspruch 7, **dadurch gekennzeichnet, daß** Papayaenzym mit 10 Gew.-%, Aloegelee mit 10 Gew.-%, Liliennaturpflanzenextrakte mit 0,5 Gew.-%, Glyzerin mit 15 Gew.-%, Mandelöl mit 15 Gew.-%, Ginsengparfüm mit 0,5 Gew.-%, Fettsäure mit 5 Gew.-%, Milchsäure mit 0,1 Gew.-%, Carbomer mit 20 Gew.-% und Wasser mit 43,9 Gew.-% vorhanden sind.

9. Arzneimittel nach Anspruch 7, **dadurch gekennzeichnet, daß** die Substrate und das kristallisierte Material mit einer Drehzahl von 1.500 Upm zwecks guter Vermischung der Substrate und des kristallinen Materials zur Bildung eines Kleisters rotiert bzw. zentrifugiert werden.
